# EUROPEAN PATENT SPECIFICATION

(11) **EP 1 622 939 B1**
(45) Date of publication and mention of the grant of the patent: **14.03.2012**
(21) Application number: 04739182.6
(22) Date of filing: 11.05.2004
(51) Int. Cl.: C07K 14/715, C07K 14/54, C07K 14/47, A61K 38/20

(54) **ACTIVE VARIANTS OF THE IL-18 BINDING PROTEIN AND MEDICAL USES THEREOF**
AKTIVE VARIANTEN DES IL-18 BINDENDEN PROTEINS UND DESSEN MEDIZINISCHE VERWENDUNGEN
VARIANTS ACTIFS DE LA PROTEINE DE LIAISON IL-18 ET UTILISATIONS MEDICALES DE CEUX-CI

(30) Priority: 13.05.2003 EP 03101326
(43) Date of publication of application: 08.02.2006
(73) Proprietor: Merck Serono SA, 1267 Coinsins, Vaud (CH)
(72) Inventor: ALTAROCCA, Valter, Marino, 00047 Rome (IT); PEZZOTTI, Anna, R., I-00181 Rome (IT)
(74) Representative: Weiss, Wolfgang
(86) International application number: PCT/EP2004/005073
(87) International publication number: WO 2004/101617

(56) References cited:
- EP-A- 1 110 969
- WO-A-02/32374
- WO-A-99/09063
- XIANG Y ET AL: "Identification of Human and Mouse Homologs of the MC51L-53L-54L Family of Secreted Glycoproteins Encoded by the Molluscum Contagiosum Poxvirus" VIROLOGY, ACADEMIC PRESS,ORLANDO, US, vol. 257, no. 2, 10 May 1999 (1999-05-10), pages 297-302, XP004439941 ISSN: 0042-6822
- YASUSHI A ET AL: "CLONING AND EXPRESSION OF INTERLEUKIN-18 BINDING PROTEIN" FEBS LETTERS, ELSEVIER SCIENCE PUBLISHERS, AMSTERDAM, NL, vol. 445, 1999, pages 338-342, XP002180254 ISSN: 0014-5793
- NOVICK D ET AL: "INTERLEUKIN-18 BINDING PROTEIN: A NOVEL MODULATOR OF THE TH1 CYTOKINE RESPONSE" IMMUNITY, CELL PRESS, US, vol. 10, no. 1, January 1999 (1999-01), pages 127-136, XP000973488 ISSN: 1074-7613 cited in the application
- KIM S-H ET AL: "STRUCTURAL REQUIREMENTS OF SIX NATURALLY OCCURRING ISOFORMS OF THE IL-18 BINDING PROTEIN TO INHIBIT IL-18" PROCEEDINGS OF THE NATIONAL ACADEMY OF SCIENCES OF USA, NATIONAL ACADEMY OF SCIENCE. WASHINGTON, US, vol. 97, no. 3, 1 February 2000 (2000-02-01), pages 1190-1195, XP000941910 ISSN: 0027-8424 cited in the application

## Description

### FIELD OF THE INVENTION

The present invention relates to new interleukin 18 binding proteins (IL-18BPs). The invention further relates to pharmaceutical compositions comprising such IL-18BPs, to nucleic acids encoding such IL-18BPs and to medical uses of said IL-18BPs.

### BACKGROUND OF THE INVENTION

In 1989, an endotoxin-induced serum activity that induced interferon-γ(IFN-γ) obtained from mouse spleen cells was described (Nakamura et al., 1989). This serum activity functioned not as a direct inducer of IFN-γ but rather as a co-stimulant together with IL-2 or mitogens. An attempt to purify the activity from post-endotoxin mouse serum revealed an apparently homogeneous 50-55 kDa protein. Since other cytokines can act as co-stimulants for IFN-γ production, the failure of neutralizing antibodies to IL-1, IL-4, IL-5, IL-6, or TNF to neutralize the serum activity suggested it was a distinct factor. In 1995, the same scientists demonstrated that the endotoxin-induced co-stimulant for IFN-γ production was present in extracts of livers from mice preconditioned with P. acnes (Okamura et al., 1995). In this model, the hepatic macrophage population (Kupffer cells) expand and in these mice, a low dose of bacterial lipopolysaccharide (LPS), which in non-preconditioned mice is not lethal, becomes lethal. The factor, named IFN-γ -inducing factor (IGIF) and later designated interleukin-18 (IL-18), was purified to homogeneity from 1,200 grams of P. acnes-treated mouse livers. Degenerate oligonucleotides derived from amino acid sequences of purified IL-18 were used to clone a murine IL-18 cDNA. IL-18 is an 18-19 kDa protein of 157 amino acids, which has no obvious similarities to any peptide in the databases. Messenger RNAs for IL-18 and interleukin-12 (IL-12) are readily detected in Kupffer cells and activated macrophages. Recombinant IL-18 induces iFN-gamma more potently than does IL-12, apparently through a separate pathway (Micallef et al., 1996). Similar to the endotoxin-induced serum activity, IL-18 does not induce IFN-γ by itself, but functions primarily as a co-stimulant with mitogens or IL-2. IL-18 enhances T cell proliferation, apparently through an IL-2-dependent pathway, and enhances Th1 cytokine production in vitro and exhibits synergism when combined with IL-12 in terms of enhanced IFN-γ production (Micallef et al., 1996).

After the murine form was cloned, the human cDNA sequence for IL-18 was reported in 1996 (Okamura et al., 1995).

By cloning IL-18 from affected tissues and studying IL-18 gene expression, a close association of this cytokine with an autoimmune disease was found. The non-obese diabetic (NOD) mouse spontaneously develops autoimmune insulitis and diabetes, which can be accelerated and synchronized by a single injection of cyclophosphamide. IL-18 mRNA was demonstrated by reverse transcriptase PCR in NOD mouse pancreas during early stages of insulitis. Levels of IL-18 mRNA increased rapidly after cyclophosphamide treatment and preceded a rise in IFN-γ mRNA, and subsequently diabetes. Interestingly, these kinetics mimic that of IL-12-p40 mRNA, resulting in a close correlation of individual mRNA levels. Cloning of the IL-18 cDNA from pancreas RNA followed by sequencing revealed identity with the IL-18 sequence cloned from Kupffer cells and in vivo pre-activated macrophages. Also NOD mouse macrophages responded to cyclophosphamide with IL-18 gene expression while macrophages from Balb/c mice treated in parallel did not. Therefore, IL-18 expression is abnormally regulated in autoimmune NOD mice and closely associated with diabetes development (Rothe et al., 1997).

IL-18 plays a potential role in immunoregulation or in inflammation by augmenting the functional activity of Fas ligand on Th1 cells (Conti et al., 1997). IL-18 is also expressed in the adrenal cortex and therefore might be a secreted neuro-immunomodulator, playing an important role in orchestrating the immune system following a stressful experience (Chater, 1986).

In vivo, IL-18 is formed by cleavage of pro-IL-18, and its endogenous activity appears to account for IFN-γ production in P. acnes and LPS-mediated lethality. Mature IL-18 is produced from its precursor by the IL-1β converting enzyme (IL-1beta-converting enzyme, ICE, caspase-1).

The IL-18 receptor consists of at least two components, co-operating in ligand binding. High- and low-affinity binding sites for IL-18 were found in murine IL-12 stimulated T cells (Yoshimoto et al., 1998), suggesting a multiple chain receptor complex. Two receptor subunits have been identified so far, both belonging to the IL-1 receptor family (Pamet et al., 1996). The signal transduction of IL-18 involves activation of NF-κB (DiDonato et al., 1997).

Recently, a soluble protein having a high affinity for IL-18 has been isolated from human urine, and the human and mouse cDNAs were described (Novick et al., 1999; WO 99/09063). The protein has been designated IL-18 binding protein (IL-18BP).

IL-18BP is not the extracellular domain of one of the known IL18 receptors, but a secreted, naturally circulating protein. It belongs to a novel family of secreted proteins. The family further includes several Poxvirus-encoded proteins which have a high homology to IL-18BP (Novick et al., 1999). IL-18BP is constitutively expressed in the spleen, belongs to the immunoglobulin superfamily, and has limited homology to the IL-1 type II receptor. Its gene was localized on human chromosome 11q13, and no exon coding for a transmembrane domain was found in an 8.3 kb genomic sequence (Novick et al., 1999).

Four human and two mouse isoforms of IL-18BP, resulting from mRNA splicing and found in various cDNA libraries and have been expressed, purified, and assessed for binding and neutralization of IL-18 biological activities (Kim et al.,"2000). Human IL-18BP *isoform a* (IL-18BPa) exhibited the greatest affinity for IL-18 with a rapid on-rate, a slow off-rate, and a dissociation constant (K(d)) of 399 pM. IL-18BPc shares the lg domain of IL-18BPa except for the 29 C-terminal amino acids; the K(d) of IL-18BPc is 10-fold less (2.94 nM). Nevertheless, IL-188Pa and IL-18BPc neutralize IL-18 >95% at a molar excess of two. IL-18BPb and IL-18BPd isoforms lack a complete lg domain and lack the ability to bind or neutralize IL-18. Murine IL-18BPc and IL-18BPd isoforms, possessing the identical lg domain, also neutralize >95% murine IL-18 at a molar excess of two. However, murine IL-18BPd, which shares a common C-terminal motif with human IL-18BPa, also neutralizes human IL-18. Molecular modeling identified a large mixed electrostatic and hydrophobic binding site in the lg domain of IL-18BP, which could account for its high affinity binding to the ligand (Kim et al., 2000).

A beneficial effect of IL-18BP in several diseases has been described. Examples of such diseases treatable with IL-18BP are: tumor metastasis (WO 01/07480), arthritis, inflammatory bowel disease and liver injury (WO 01/62285), heart disease (WO 02/060479), traumatic brain injury (WO 02/096456), sepsis (WO 021092008), atherosclerosis (WO 01/85201), hypersensitivity disorder (WO03/033015).

An IL-18 binding domain of a viral homologue of human IL-18BP has been described in the literature (Xiang and Moss, 2003), showing that a furin cleaved form of the IL-18 binding protein of Molluscum contagiosum virus has IL-18 binding properties. In addition to this, several point mutations were introduced into the viral homologue of IL-18BP and tested in vitro for IL-18 binding in order to define the biologically active portions of the protein (Xiang and Moss, 2001).

However, biologically active fragments of the human IL-18BP *isoform a* have not been identified and characterized so far.

### SUMMARY OF THE INVENTION

The present invention is based on the finding that during production of recombinant human IL-18BP *isoform a*, variant forms can be found that retain the biological activity of IL-18BP, as measured in an in vitro bioassay. The invention therefore relates to variants of IL-18BP as defined in the attached claims in which an internal clipping of IL-18BP has occurred.

Such variants represent active variants of the mature IL-18BP.

Described are also nucleic acid molecules coding for such IL-18BP variants, to pharmaceutical compositions comprising these variants. The invention relates to the use of such nucleic acids for the preparation of medicaments for the treatment and/or prevention of IL-18 mediated disorders.

In a further aspect, the invention relates to the use of an expression vector comprising the coding sequence of an IL-18BP variant for the treatment and/or prevention of IL-18 mediated diseases.

The invention further relates to processes of production of the IL-18BP variants of the invention.

### BRIEF DESCRIPTION OF THE DRAWINGS

Fig. 1 shows the sequence of full-length IL-18BP *isoform a*. Putative N-glycosylation sites are labeled as N*. Arrows mark the N-termini of the six IL-18BP variants of the invention.
Fig. 2 shows a silver stained SDS-PAGE gel (A) and the corresponding western blot (B) of an IL-18BP preparation containing IL-18BP variants of the invention. The lanes were loaded as follows:

| Fig. 2 A: | | Fig. 2B | |
|---|---|---|---|
| | 1. MW marker | | 1. MW marker |
| | 2. r-hIL-18BP CT20 (2 µg) | | 2. r-hIL-18BP CT20 (200 ng) |
| | 3. r-hIL-18BP CT20 (2 µg) | | 3. ST1P01/r-hIL-18BP (200 ng) |
| | 4. r-hIL-18BP CT20 (2 µg) | | |
| | 5. ST1P01/r-hIL-18BP | | 4. MW marker |

Fig. 3. shows the SE-HPLC profile as well as silver stained SDS-PAGE gel (A) and the corresponding western blot (B) of the two peaks obtained in HPLC as compared to a standard preparation of pure full-length IL-18BP *isoform a*.

### DESCRIPTION OF THE INVENTION

The present invention is based on the finding that variants of IL-18BP could be identified during recombinant production of human recombinant IL-18BP *isoform a*. These variants were characterized and it was found that they represent defined N-and C-terminally truncated fragments of full-length IL-18BP *isoform a*. Definition of the N-glycosylation pattern of recombinant IL-18BP could be achieved in the frame of the present invention, leading to a new variant of full-length IL-18BP.

Surprisingly, all variants of IL-18BP displayed a biological activity comparable to full-length IL-18BP in an *in vitro* bioassay.

In these variants of IL-18BP, an internal clipping of IL-18BP has occurred.

Thus the invention relates to an IL-18BP comprising a first polypeptide consisting of amino acids 1 to 30 of SEQ ID NO: 1 and a second polypeptide consisting of amino acids 31 to 164 or of amino acids 31 to 163, wherein the first and second polypeptide are linked by a disulfide bond.

In a second aspect, the IL-18BP comprises a first polypeptide consisting of amino acids 15 to 30 of SEQ ID NO: 1 and a second polypeptide consisting of amino acids 31 to 164 or of amino acids 31 to 163, wherein the first and second polypeptide are linked by a disulfide bond.

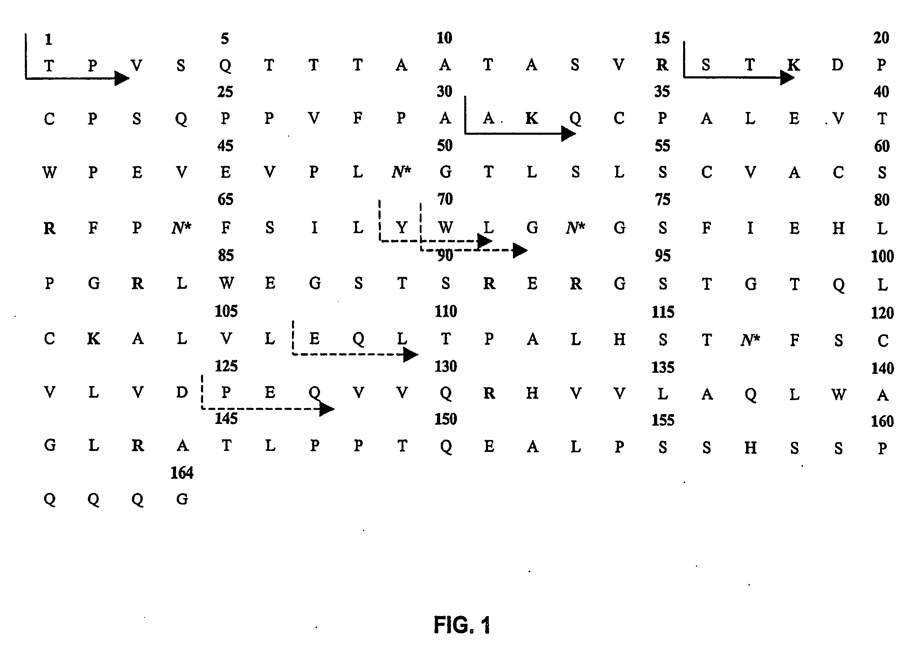

The IL-18BPs may be unglycosylated or glycosylated. Preferably, the IL-18BPs of the invention are N-glycosylated at asparagine residues Asn 49, Asn 73 and Asn 117 (numbering according to Fig. 1).

During the experiments leading to the present invention, it has been found for the first time that recombinantly produced full-length IL-18 binding protein *isoform* a is not glycosylated at all putative N-glycosylation sites, but only at three defined Asparagine residues, which are Asn 49, 73 and 117. Therefore, there is disclosed an IL-18BP having the amino acid sequence of SEQ ID NO: 1, wherein the protein is N-glycosylated at Asn 49, Asn 73 and Asn 117.

The sequence of full length human IL-18BP and its splice variants/isoform are disclosed e.g. from WO99/09063, or from Novick et al., 1999, as well as in Kim et al., 2000. SEQ ID NO: 1 represents the amino acid sequence of mature full-length IL-18BP *isoform a*.

In the following, the proteins of the invention may be generally designated "IL-18BP", "IL-18BPs" or "IL-18BP(s) of the invention". These terms, as used therein, encompass all IL-18BP variants described in the frame of the present invention.

The proteins according to the present invention may be derived from natural sources, such as urine, or they may preferably be produced recombinantly. Recombinant expression may be carried out in prokaryotic expression systems like E. coli, or in eukaryotic, and preferably in mammalian, expression systems. They may also preferably be produced in human expression systems. Established cell lines such as the Chinese hamster ovary cell line (CHO) or the human embryonic kidney cell line 293 may be especially useful for production of the IL-18BP variants of the present invention.

Further variants within the scope of the present invention may be proteins having conservative amino acid substitutions of the sequences depicted in Fig. 1 or the annexed sequence listing. These variants may be prepared by known synthesis and/or by site-directed mutagenesis techniques, or any other known technique suitable therefor.

Conservative amino acid substitutions of IL-18BP polypeptides, may include synonymous amino acids within a group which have sufficiently similar physicochemical properties that substitution between members of the group will preserve the biological function of the molecule (Grantham, 1974). It is clear that insertions and deletions of amino acids may also be made in the above-defined sequences without altering their function, particularly if the insertions or deletions only involve a few amino acids, e.g., under thirty, and preferably under ten, and do not remove or displace amino acids which are critical to a functional conformation, e.g., cysteine residues. Proteins and muteins produced by such deletions and/or insertions come within the purview of the present invention.

Preferably, the synonymous amino acid groups are those defined in Table 1. More preferably, the synonymous amino acid groups are those defined in Table 2; and most preferably the synonymous amino acid groups are those defined in Table 3.

**TABLE 1**

| Preferred Groups of Synonymous Amino Acids | |
|---|---|
| Amino Acid | Synonymous Group |
| Ser | Ser, Thr, Gly, Asn |
| Arg | Arg, Gln, Lys, Glu, His |
| Leu | Ile, Phe, Tyr, Met, Val, Leu |
| Pro | Gly, Ala, Thr, Pro |
| Thr | Pro, Ser, Ala, Gly, His, Gin, Thr |
| Ala | Gly, Thr, Pro, Ala |
| Val | Met, Tyr, Phe, Ile, Leu, Val |
| Gly | Ala, Thr, Pro, Ser, Gly |
| Ile | Met, Tyr, Phe, Val, Leu, Ile |
| Phe | Trp, Met, Tyr, Ile, Val, Leu, Phe |
| Tyr | Trp, Met, Phe, Ile, Val, Leu, Tyr |
| Cys | Ser, Thr, Cys |
| His | Glu, Lys, Gln, Thr, Arg, His |
| Gln | Glu, Lys, Asn, His, Thr, Arg, Gin |
| Asn | Gln, Asp, Ser, Asn |
| Lys | Glu, Gin, His, Arg, Lys |
| Asp | Glu, Asn, Asp |
| Glu | Asp, Lys, Asn, Gln, His, Arg, Glu |
| Met | Phe, Ile, Val, Leu, Met |
| Trp | Trp |

**TABLE 2**

| More Preferred Groups of Synonymous Amino Acids | |
|---|---|
| Amino Acid | Synonymous Group |
| Ser | Ser |
| Arg | His, Lys, Arg |
| Leu | Leu, Ile, Phe, Met |
| Pro | Ala, Pro |
| Thr | Thr |
| Ala | Pro, Ala |
| Val | Val, Met, Ile |
| Gly | Gly |
| Ile | Ile, Met, Phe, Val, Leu |
| Phe | Met, Tyr, Ile, Leu, Phe |
| Tyr | Phe, Tyr |
| Cys | Cys, Ser |
| His | His, Gln, Arg |
| Gln | Glu, Gln, His |
| Asn | Asp, Asn |
| Lys | Lys, Arg |
| Asp | Asp, Asn |
| Glu | Glu, Gln |
| Met | Met, Phe, Ile, Val, Leu |
| Trp | Trp |

**TABLE 3**

| Most Preferred Groups of Synonymous Amino Acids | |
|---|---|
| Amino Acid | Synonymous Group |
| Ser | Ser |
| Arg | Arg |
| Leu | Leu, Ile, Met |
| Pro | Pro |
| Thr | Thr |
| Ala | Ala |
| Val | Val |
| Gly | Gly |
| Ile | Ile, Met, Leu |
| Phe | Phe |
| Tyr | Tyr |
| Cys | Cys, Ser |
| His | His |
| Gln | Gln |
| Asn | Asn |
| Lys | Lys |
| Asp | Asp |
| Glu | Glu |
| Met | Met, Ile, Leu |
| Trp | Met |

It is understood that minor changes in the amino acid sequence of the IL-18BP variants are within the scope of the invention, having a sequence of amino acids sufficiently duplicative of that of an IL-18BP variant described herein, such as to have a comparable activity to IL-18BP. One activity of IL-18BP is its capability of binding IL-18. Thus, it can be determined whether any given variant has substantially the same activity as IL-18BP by means of routine experimentation comprising subjecting such a mutein, e.g., to a simple sandwich competition assay to determine whether or not it binds to an appropriately labeled IL-18, such as radio-immunoassay or ELISA assay. A further meaningful assay describing IL-18BP activity is the bioassay described in the example below.

Examples of production of amino acid substitutions in proteins which can be used for obtaining variants of IL-18BP polypeptides or proteins for use in the present invention include any known method steps, such as presented in US patents 4,959,314, 4,588,585 and 4,737,462, to Mark et al; 5,116,943 to Koths et al., 4,965,195 to Namen et al; 4,879,111 to Chong et al; and 5,017,691 to Lee et al; and lysine substituted proteins presented in US patent No. 4,904,584 (Shaw et al).

In an embodiment of the invention, the IL-18BP variants are fused proteins.

The term "fused protein" refers to a polypeptide comprising an IL-18BP of the invention, fused with another protein, which, e.g., has an extended residence time in body fluids. An IL-18BP may thus be fused to another protein, polypeptide or the like, e.g., an immunoglobulin or a fragment thereof.

In a preferred embodiment of the invention, the IL -18BP of the invention comprises an immunoglobulin fusion, i.e. it is a fused protein comprising all or part of an IL-18BP of the invention, which is fused to all or a portion of an immunoglobulin. Methods for making immunoglobulin fusion proteins are well known in the art, such as the ones described in WO 01/03737, for example. The person skilled in the art will understand that the resulting fusion protein of the invention retains the biological activity of IL-18BP, in particular the binding to IL-18. The fusion may be direct, or via a short linker peptide which can be as short as 1 to 3 amino acid residues in length or longer, for example, 13 amino acid residues in length. Said linker may be a tripeptide of the sequence E-F-M (Glu-Phe-Met), for example, or a 13-amino acid linker sequence comprising Glu-Phe-Gly-Ala-Gly-Leu-Val-Leu-Gly-Gly-Gln-Phe-Met introduced between the IL-188P sequence and the immunoglobulin sequence. The resulting fusion protein has improved properties, such as an extended residence time in body fluids (half-life), increased specific activity, increased expression level, or the purification of the fusion protein may be facilitated.

In a preferred embodiment, IL-18BP is fused to the constant region of an lg molecule. Preferably, it is fused to heavy chain regions, like the CH2 and CH3 domains of human IgG1 or IgG3, for example. The generation of specific fusion proteins comprising IL-18BP and a portion of an immunoglobulin are described in example 11 of WO 99/09063, for example. Other isoforms of Ig molecules are also suitable for the generation of fusion proteins according to the present invention, such as isoforms IgG₂ or IgG₄, or other Ig classes, like IgM or IgA, for example. Fusion proteins may be monomeric or multimeric, hetero- or homomultimeric.

The invention further relates to a process for production of an IL-18BP fused protein comprising preparing a DNA construct that encodes an IL-18BP of the invention ligated to a nucleic acid encoding a second polypeptide, wherein upon expression, said DNA construct encodes a fusion protein comprising the IL-18BP of the invention fused to the second polypeptide.

Preferably, the second polypeptide is a portion of an immunoglobulin, more preferably the Fc portion of an immunoglobulin.

In a further embodiment, the IL-18BP variants are functional derivatives, wherein the functional derivative comprises at least one moiety attached to one or more functional groups, which occur as one or more side chains on the amino add residues.

"Functional derivatives" as used herein cover derivatives of IL-18BP variants or their fused proteins, which may be prepared from the functional groups which occur as side chains on the residues or the N- or C-terminal groups, by means known in the art, and are included in the invention as long as they remain pharmaceutically acceptable, i.e. they do not destroy the activity of the protein which is substantially similar to the activity of IL-18BP, or viral IL-18BPs, and do not confer toxic properties on compositions containing it. Functional derivatives of IL-18BP may be conjugated to polymers in order to improve the properties of the protein, such as the stability, half-life, bioavailability, tolerance by the human body, or immunogenicity. To achieve this goal, IL18-BP may be linked e.g. to Polyethlyenglycol (PEG). PEGylation may be carried out by known methods, described in WO 92/13095, for example.

Derivatives may also, for example, include aliphatic esters of the carboxyl groups, amides of the carboxyl groups by reaction with ammonia or With primary or secondary amines, N-acyl derivatives of free amino groups of the amino acid residues formed with acyl moieties (e.g. alkanoyl or carbocyclic aroyl groups) or O-acryl derivatives of free hydroxyl groups (for example that of seryl or threonyl residues) formed with acyl moieties.

The invention further relates to a process for production of an IL-18BP derivative of the invention comprising chemically modifying an IL-18BP of the invention to include at least one derivative moiety. Preferably, the moiety is a polyethylene glycol moiety.

Yet a further embodiment of the invention relates to salts of the IL-18BP variants.

The term "salts" herein refers to both salts of carboxyl groups and to acid addition salts of amino groups of IL-18BP variant molecule, or analogs thereof. Salts of a carboxyl group may be formed by means known in the art and include inorganic salts, for example, sodium, calcium, ammonium, ferric or zinc salts, and the like, and salts with organic bases as those formed, for example, with amines, such as triethanolamine, arginine or lysine, piperidine, procaine and the like. Acid addition salts include, for example, salts with mineral acids, such as, for example, hydrochloric acid or sulfuric acid, and salts with organic acids, such as, for example, acetic acid or oxalic acid. Of course, any such salts must retain the biological activity of the IL-18BP relevant to the present invention, such as inhibition of IFN-gamma induction in the bioassay described in the examples below.

Described is further a nucleic acid coding for an IL-18BP of the invention. Such coding sequence may easily be deduced from the amino acid sequences depicted in Fig. 1 or the annexed sequence listing. The person skilled in the art will appreciate that many more nucleic acid sequences coding for the IL-18BPs of the invention can be conceived due to the degeneracy of the genetic code.

Described is further a host cell comprising the nucleic acid of the invention. Such a host cell may be either prokaryotic or eukaryotic, preferably mammalian, more preferably a host cell suitable for recombinant expression of therapeutic proteins such as Chinese hamster ovary cells (CHO) or human cells.

The invention further relates to a process for production of an IL-18BP of the invention comprising the step of culturing a host cell as described under conditions suitable for expression of said IL-18BP.

The process for production of an IL-18BP may also comprise the step of isolating the IL-18BP from the cell culture supernatant of a host cell of the invention.

In another aspect, the invention relates to a composition comprising an IL-18BP in accordance with the present invention. Preferably, it is a pharmaceutical composition. Optionally, the pharmaceutical composition further comprises pharmaceutically acceptable surfactants, excipients, carriers, diluents and vehicles.

The definition of "pharmaceutically acceptable" is meant to encompass any carrier, which does not interfere with effectiveness of the biological activity of the active ingredient and that is not toxic to the host to which it is administered. For example, for parenteral administration, the active protein(s) may be formulated in a unit dosage form for injection in vehicles such as saline, dextrose solution, serum albumin and Ringer's solution.

The active ingredients of the pharmaceutical composition according to the invention can be administered to an individual in a variety of ways. The routes of administration include intradermal, transdermal (e.g. in slow release formulations), intramuscular, intraperitoneal, intravenous, subcutaneous, oral, intracranial, epidural, topical, rectal, and intranasal routes.

Preferred administration routes of the invention are the subcutaneous and the intramuscular route.

Any other therapeutically efficacious route of administration can be used, for example absorption through epithelial or endothelial tissues, or by gene therapy wherein a DNA molecule encoding the active agent is administered to the patient (e.g. via a vector), which causes the active agent to be expressed and secreted in vivo. If an expression vector comprising the coding sequence of IL-18BP(s) of the invention is to be administered, it may e.g. be injected intramuscularly as naked DNA.

For parenteral (e.g. intravenous, subcutaneous, intramuscular) administration, the active protein(s) can be formulated as a solution, suspension, emulsion or lyophilized powder in association with a pharmaceutically acceptable parenteral vehicle (e.g. water, saline, dextrose solution) and additives that maintain isotonicity (e.g. mannitol) or chemical stability (e.g. preservatives and buffers). The formulation is sterilized by commonly used techniques.

The bioavailability of the active protein(s) according to the invention can also be ameliorated by using conjugation procedures which increase the half-life of the molecule in the human body, for example linking the molecule to polyethylene glycol, as described in the PCT Patent Application WO 92/13095.

The therapeutically effective amounts of the active protein(s) will be a function of many variables, including the type of IL-18BP use, their affinity for IL-18, any residual cytotoxic activity exhibited by the IL-18BP(s), the route of administration, the clinical condition of the patient (including the desirability of maintaining a non-toxic level of endogenous IL-18 activity).

A "therapeutically effective amount" is such that when administered, the IL-18BP variant results in inhibition of the biological activity of IL-18. The dosage administered, as single or multiple doses, to an individual will vary depending upon a variety of factors, including IL-18BP variant pharmacokinetic properties, the route of administration, patient conditions and characteristics (sex, age, body weight, health, size), extent of symptoms, concurrent treatments, frequency of treatment and the effect desired. Adjustment and manipulation of established dosage ranges are well within the ability of those skilled in the art, as well as in vitro and in vivo methods of determining the inhibition of IL-18 in an individual.

In a preferred embodiment of the present invention, the IL-18BP variant is used in an amount of about 0.001 to 1000 mg/kg of body weight, or about 0.001 to 100 mg/kg of body weight or about 0.01 to 10 mg/kg of body weight or about 0.1 to 5 mg/kg or about 1 to 3 mg/kg of body weight.

The frequency of administration may be daily or every other day. It may also be three times per week or once per week.

The doses administered may always be the same or vary, depending on the patient's needs. The doses are usually given in divided doses or in sustained release form effective to obtain the desired results. Second or subsequent administrations can be performed at a dosage which is the same, less than or greater than the initial or previous dose administered to the individual. A second or subsequent administration can be administered during or prior to onset of the disease.

According to the invention, the IL-18BP variant can be administered prophylactically or therapeutically to an individual prior to, simultaneously or sequentially with other therapeutic regimens or agents (e.g. multiple drug regimens), in a therapeutically effective amount, in particular with an interferon and/or a TNF inhibitor. Active agents that are administered simultaneously with other therapeutic agents can be administered in the same or different compositions.

In a further aspect, the invention relates to the use of an IL-18BP of the invention for the preparation of a medicament for treatment and/or prevention of an IL-18 mediated disease or disorder. IL-18 mediated diseases are known in the art (reviewed e.g. by Gracie et al., 2003).

In a preferred embodiment, the disease to be treated or prevented by the IL-18P variant of the invention is selected from psoriasis, arthritis, in particular rheumatoid arthritis, inflammatory bowel disease, in particular Crohn's disease, liver injury, atherosclerosis, sepsis, myocardial infarction, traumatic brain injury, allergy, peripheral vascular disease, multiple sclerosis, tumor metastasis.

For detailed description and definition of these diseases, it is particularly referred to the following published patent applications:
WO 99/09063, WO 01/07480, WO 01/62285. WO 02/060479, WO 02/096456, WO 02/092008. WO 03/013577.

Interferons are predominantly known for inhibitory effects on viral replication and cellular proliferation, interferon-γ, for example, plays an important role in promoting immune and inflammatory responses. Interferon β (IFN-β, an interferon type I), is said to play an anti-inflammatory role.

The invention therefore also relates to the use of a combination of an IL-18BP of the invention and an interferon in the manufacture of a medicament for the treatment of an IL-18 mediated disease.

Interferons may also be conjugated to polymers in order to improve the stability of the proteins. A conjugate between interferon β and the polyol Polyethlyenglycol (PEG) has been described in WO99/55377, for instance.

In another preferred embodiment of the invention, the interferon is Interferon-β (IFN-β), and more preferably IFN-β 1a.

The IL-18BP of the invention is preferably used simultaneously, sequentially, or separately with the interferon.

In yet a further embodiment of the invention, an IL-18BP of the invention is used in combination with a TNF antagonist. TNF antagonists exert their activity in several ways. First, antagonists can bind to or sequester the TNF molecule itself with sufficient affinity and specificity to partially or substantially neutralize the TNF epitope or epitopes responsible for TNF receptor binding (hereinafter termed "sequestering antagonists"). A sequestering antagonist may be, for example, an antibody directed against TNF.

Alternatively, TNF antagonists can inhibit the TNF signaling pathway activated by the cell surface receptor after TNF binding (hereinafter termed "signaling antagonists"). Both groups of antagonists are useful, either alone or together, in combination with an IL-18BP variant, in the therapy of hypersensitivity disorders.

TNF antagonists are easily identified and evaluated by routine screening of candidates for their effect on the activity of native TNF on susceptible cell lines in vitro, for example human B cells, in which TNF causes proliferation and immunoglobulin secretion. The assay contains TNF formulation at varying dilutions of candidate antagonist, e.g. from 0.1 to 100 times the molar amount of TNF used in the assay, and controls with no TNF or only antagonist (Tucci et al., 1992).

Sequestering antagonists are the preferred TNF antagonists to be used according to the present invention. Amongst sequestering antagonists, those polypeptides that bind TNF with high affinity and possess low immunogenicity are preferred. Soluble TNF receptor molecules and neutralizing antibodies to TNF are particularly preferred. For example, soluble TNF-RI (also called p55) and TNF-RII (also called p75) are useful in the present invention. Truncated forms of these receptors, comprising the extracellular domains of the receptors or functional portions thereof, are more particularly preferred antagonists according to the present invention. Soluble TNF type-I and type-II receptors are described in European Patents EP 308 378, EP 398 327 and EP 433 900, for example.

These truncated, soluble TNF receptors are soluble and have been detected in urine and serum as TNF inhibitory binding proteins, called TBPI and TBPII, respectively (Engelmann et al., 1990). The simultaneous, sequential, or separate use of the IL-18BP variant with the TNF antagonist and /or an Interferon is preferred, according to the invention.

According to the invention, TBP I and TBPII are preferred TNF antagonists to be used in combination with an IL-18BP variant of the invention. Derivatives, fragments, regions and biologically active portions of the receptor molecules functionally resemble the receptor molecules that can also be used in the present invention. Such biologically active equivalent or derivative of the receptor molecule refers to the portion of the polypeptide, or of the sequence encoding the receptor molecule, that is of sufficient size and able to bind TNF with such an affinity that the interaction with the membrane-bound TNF receptor is inhibited or blocked.

The invention further relates to the use of an expression vector comprising the coding sequence of an IL-18BP of the invention in the preparation of a medicament for the prevention and/or treatment of IL-18 meditated disorders. Thus, a gene therapy approach is considered in order to deliver the IL-18BP variant to the site where it is required. In order to treat and/or prevent a hypersensitivity disorder, the gene therapy vector comprising the sequence of an IL-18BP variant production and/or action may be injected directly into the diseased tissue, for example, thus avoiding problems involved in systemic administration of gene therapy vectors, like dilution of the vectors, reaching and targeting of the target cells or tissues, and of side effects.

The invention further relates to the use of a cell that has been genetically modified to produce an IL-18BP of the invention in the manufacture of a medicament for the treatment and/or prevention of an IL-18 mediated disease.

The invention further relates to a method for the preparation of a pharmaceutical composition comprising admixing an effective amount of an IL-18BP variant and/or an interferon and/or a TNF antagonist with a pharmaceutically acceptable carrier.

The invention further relates to a method of treatment of IL-18 mediated disease, comprising administering a pharmaceutically effective amount of an IL-18BP variant to a patient In need thereof.

Having now fully described this invention, it will be appreciated by those skilled in the art that the same can be performed within a wide range of equivalent parameters, concentrations and conditions without departing from the spirit and scope of the invention and without undue experimentation.

While this invention has been described in connection with specific embodiments thereof, it will be understood that it is capable of further modifications. This application is intended to cover any variations, uses or adaptations of the invention following, in general, the principles of the invention and including such departures from the present disclosure as come within known or customary practice within the art to which the invention pertains and as may be applied to the essential features hereinbefore set forth as follows in the scope of the appended claims.

Reference to known method steps, conventional methods steps, known methods or conventional methods is not any way an admission that any aspect, description or embodiment of the present invention is disclosed, taught or suggested in the relevant art.

The foregoing description of the specific embodiments will so fully reveal the general nature of the invention that others can, by applying knowledge within the skill of the art (including the contents of the references cited herein), readily modify and/or adapt for various application such specific embodiments, without undue experimentation, without departing from the general concept of the present invention. Therefore, such adaptations and modifications are intended to be within the meaning an range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art

### EXAMPLE

### IDENTIFICATION OF IL-18BP VARIANTS

| MATERIALS and METHODS | |
|---|---|
| **Materials and equipment** | |
| 96 well microtiter plate photometer MCC 349 or EX | Labsystem |
| Analytical balance mod. AG145 | Mettler-Toledo |
| Aquapore RP300 30 x 4.6 mm cartridge cod. 0711-0055 | Brownlee |
| Automated sequencer mod. Procise 494 | Applied Biosystem |
| Automatic pipettes (P1000, P200, P100, P20) | Gilson |
| Cell Coulter | Counter -Z1 |
| CO₂ incubator | Heraeus |
| Excel software | |
| Freezer -20°C ± 5°C | Angelantoni |
| Freezer -80°C ± 10°C | Angelantoni |
| Graph Pad Prism Software | |
| HPLC mod. Alliance 2690 | Waters |
| HPLC-pump mod. 600S with column heater | Waters |
| Integrator D2500 | Merck |
| Laminar Flow Hood | Flow Laboratories |
| MALDI-ToF mod. Voyager DE-Pro | Perseptive Biosystem |
| Mass Spectrometer mod. ZQ | Waters Micromass |
| Multiphor II | Pharmacia |
| Multitemp II | Pharmacia or equivalent |
| Personal computer | CompaQ |
| pH meter MA235 | Mettler or equivalent |
| pH-meter mod MP225 | Mettler-Toledo |
| Power supply EPS 3501 XL | Pharmacia or equivalent |
| Refrigerator +5°C ± 3°C | Angelantoni |
| Scanner AGFA Arcus II | Agfa or equivalent |
| Separation module 2690 Alliance | Waters |
| Software Agfa Fotolook v.3.0 | Agfa or equivalent |
| Software Millennium³² version 3.20 | Waters |
| Software Phoretix 1 D | Phoretix or equivalent |
| Software Picture Publisher v.8 | Micrografx or equivalent |
| Spectrolinker XL 1000 Cross Linker (UV source) | Spectronics Corporation |
| Statgraphics Plus Symmetry C18 3.5 µm 75 x 4,6 mm column cod. WAT066224 Waters | |
| Technical balance mod PEG2002 | Mettler-Toledo |
| UV detector 2487 | Waters |
| UV detector mod. 2487 | Waters |
| UV detector mod. 996 | Waters |
| | |
| *Chemicals* | |
| Dithiothreitol (DTT) cod. D5545 | Sigma |
| Tris cod.1.08382 | Merck |
| EDTA cod.1.08418 | Merck |
| Acetonitrile (ACN) cod. 1.00030 | Merck |
| Ammonium bicarbonate cod. 1.01131 | Merck |
| Ammonia 25% cod. 1.05432 | Merck |
| Calcium chloride 2 H₂O cod. 13381 | Sigma |
| Endoproteinase Bovine Trypsin, Modified, sequencing grade cod. 1418-025 | Roche |
| Neuraminidase (Sialidase) cod. 1080-725 | Roche |
| Water (H₂O) MilliQ Grade | Millipore |
| Trifluoroacetic acid (TFA) cod. 9470 | Baker |
| Acetic acid glacial cod. 00063 | Merck |
| Sodium Hydroxide cod. 7067 | Baker |
| Iodoacetic acid cod. 12512 | Sigma |
| Sodium acetate 3M pH 5.5 cod. 400471 | Applied Biosystem |
| Hydrochloric acid 37% cod.1.000314 | Merck |
| β-Mercaptoethanol cod. M 6250 | Sigma |
| Diethylether cod. cod 447521 | Carlo Erba |
| Guanidine cod. N24115 | Pierce |
| NaCsl cod. 700000889-2 | ULTRA Scientific |
| Nitrogen UPP | Caracciolo |
| Methanol gradient grade cod 1.06007 | Merck |
| Eppendorf 1.5 ml | Eppendorf |
| Water (H₂O) purified by Modulab 2020™ | Continental |
| Acetonitrile (HPLC grade) code 1.00030 | Merck |
| *o*-Phoshoric acid (H₃PO₄) 85% code 1.00573 | Merck |
| Sodium sulfate (Na₂SO₄) code 1.06649 | Merck |
| Column TSK G2000 SW_{XL} 7.8x300 code 08540 | TosoHaas |
| Goat anti mouse IgG HRP conjugated cod. 170-6516 | BioRad |
| Monoclonal antibody anti r-hIL-18BP clone 582.10 | IPL |
| ExcelGel SDS buffer strips cod. 17-1342-01 | Pharmacia |
| ExcelGel SDS Homogeneous 12.5% cod. 80-1261-01 | Pharmacia |
| Hyperfilm ECL 18 x 24 cm cod. RPN 2103 | Pharmacia-Biotech |
| Kit ECL cod. RPN2106 | Pharmacia-Biotech |
| Kit Silver PlusOne cod. 17-1150-01 | Pharmacia |
| Nitrocellulose membrane 0.2mm cod. BA-83 | Schleicher & Schuell |
| I-Block cod. Al 300 | Tropix |
| Interim Reference Material ST1P01/r-hIL-18BP | IFS |
| Molecular weight marker (97-14 kDa) cod. 17-0446-01 | Pharmacia |
| Tween 20 | Merck |
| Phosphate buffered saline (PBS). with calcium and magnesium ions. | Sigma |
| 96 wells plate | Falcon |
| 96 wells microtiter plate | Maxi Sorp Nunc |
| IMDM | GIBCO |
| 2-Mercaptoethanol | Sigma |
| Penicilin/Streptomycin | Gibco |
| Foetal Bovine Serum (FBS) | GIBCO |
| Human IFN-γ Immunoassay Kit - DUO Set ELISA Development System | R&D Systems |
| Bovine Serum Albumin (BSA) | Sigma |
| Substrate solution | R&D Systems |
| Sulphuric Acid (H₂SO₄) | Merck |
| | |
| *Biologicals* | |
| Human acute myelogenous leukemia cell line KG-1 | In house |
| Recombinant human Tumor Necrosis Factor-alpha (TNF-α) | R&D Systems |
| Recombinant human Interleukin-18 (r-hIL-18) | Produced in house |
| Recombinant human Interleukin-18 Binding Protein-(r-hIL-18BP) | produced in house |
| (46.39 mg/ml by Amino Acid Analysis) | |

### METHODS

### PEPTIDE MAPPING BY TRYPSIN

The mapping was carried out according to standard protocols, outlined below.

### TREATMENT WITH NEURAMINIDASE

About 150µg of dried r-hIL-18BP was dissolved with 200µL of 0.2M Ammonium Acetate 16mM Calcium Chloride pH 5.5 buffer and 100mlU of Sialidase. The reaction was performed at 37°±1° C for 1 hour. Then the protein was dried in Speed-Vacuum. After desiccation the protein was reduced and alkylated as described below.

### REDUCTION AND ALKYLATION

Dissolved with 200µL of 0.5M Tris-Cl 2mM EDTA pH 8,5±0.05 6M Guanidine 11 mg/mL dithiotreitol under nitrogen atmosphere. The reaction was performed at room temperature for 1 hour. Then has been added 25µL of 250mg/mL iodioacetic acid under nitrogen atmosphere. The mixture was incubated in the dark at 37±1° °C for 45 minutes and then stopped by adding 200µL of 0.1% aqueous TFA and 20µL β-mercaptoethanol under nitrogen atmosphere .The reaction was incubated at room temperature for 15 minutes.

### PURIFICATION PROCEDURE

After reduction and alkylation, the protein was purified in RP-HPLC as described below:

| | |
|---|---|
| Column: | Aquapore RP 300 (4.6x30 mm) cod. 0711-0055 Brownlee |
| Eluent A: | 0.1 % aqueous TFA |
| Eluent B: | 0.1 % TFA in CH₃CN |
| Column Temperature: | + 40°C |
| UV detector set at 214 nm | |

| **Gradient** | | | | |
|---|---|---|---|---|
| **Time (minutes)** | **Flow (ml/min)** | **%A** | **%B** | **Curve** |
| 0 | 1 | 95 | 5 | |
| 5 | 1 | 95 | 5 | 6 |
| 6 | 1 | 80 | 20 | 6 |
| 41 | 1 | 35 | 65 | 6 |
| 46 | 1 | 20 | 80 | 1 |
| 47 | 1 | 95 | 5 | 6 |
| 57 | 1 | 95 | 5 | 6 |

The purified material was dried in speed-vac, dissolved in 250 µL of 0.1M ammonium bicarbonate pH 9,0 ± 0.05 and incubated with 5µL of modified bovine trypsin at 37 ± 1°C for 4 hours with intermittent shaking. The reaction was stopped by adding 60µL of 5% aqueous TFA.

### ANALYTICAL RP-HPLC OF TRYPTIC PEPTIDE MAPPING

Half volume of the r-hlL-18BP peptide mixture was purified in RP-HPLC as described below:

| | |
|---|---|
| Column: | Waters Symmetry C18 3,5µm (4.6x75 mm) |
| Eluent A: | 0.1 % aqueous TFA |
| Eluent B: | 0.1 % TFA in CH₃CN |
| Temperature: | +45°C |
| UV detector set at 214 nm | |

| **GRADIENT** | | | | |
|---|---|---|---|---|
| **Time (minutes)** | **Flow (ml/min)** | **%A** | **%B** | **Curve** |
| 0 | 1.0 | 98 | 2 | |
| 2 | 1.0 | 98 | 2 | 6 |
| 61 | 1.0 | 57 | 43 | 6 |
| 63 | 1.0 | 10 | 90 | 1 |
| 65 | 1.0 | 98 | 2 | 6 |

### EDMAN SEQUENCING ANALYSIS

Automated Edman sequencing was carried out on a Procise protein sequencer, according to the manufacturer's instructions.

### MALDI-TOF

MALDI-ToF spectra were carried out on a Voyager PE-Pro, according to manufacturer instructions.

### LC-ES/MS OF TRYPSIN PEPTIDE MAPPING

The r-hIL-18BP was submitted to the peptide mapping procedure following the procedure mentioned above. After the digestion an aliquot of the peptide mixture of each sample was analysed as described below:

| | |
|---|---|
| Column: | Waters Symmetry C18 3,5µm (4.6x75 mm) |
| Eluent A: | 0.1 % aqueous TFA |
| Eluent B: | 0.1 % TFA in CH₃CN |
| Temperature: | +45°C |
| UV detector set at 214 nm | |

| **Gradient** | | | | |
|---|---|---|---|---|
| **Time (minutes)** | **Flow (ml/min)** | **%A** | **%B** | **Curve** |
| 0 | 0.7 | 98 | 2 | |
| 12 | 0.7 | 98 | 2 | 6 |
| 71 | 0.7 | 57 | 43 | 6 |
| 73 | 0.7 | 10 | 90 | 1 |
| 75 | 0.7 | 98 | 2 | 6 |

After UV detector the flow was split in order to introduce in the spectrometer source at 50µL/min.

The mass spectrometer has been set with the following parameters:

| | |
|---|---|
| Capillary voltage: | 3.5 KV |
| Cone voltage: | 35 V |
| HV lenses: | 0.45 KV |
| Source temperature: | 80°C |
| Resolution: | 14 HM; 14 LM |

### SEC SELECTED METHOD FOR DIMERS/AGGREGATES CONTENT

The SE-HPLC analyswas was carried out as reported below:

| | |
|---|---|
| Eluent | 0.1 M H₃PO₄, 0.3 M Na₂SO₄, pH 7.3 with NaOH, CAN 3% |
| Column type | TSK G2000 SW_{XL} 7.8x300 code 08540 |
| Autosampler temperature | + 4°C±2°C |
| Column temperature | Room temperature |
| Detection wavelength | 214 nm |
| Flow rate of mobile phase | 0.5 mL/min |
| Analysis time | 30 minutes. |
| Delay for next injection | Not less than 5 minutes |

### SDS-PAGE AND SILVER STAINING

Two micrograms of r-hIL18BP were loaded onto the precast gel ExcelGel^{®} SDS Homogeneous 12.5% (by Amersham Biosciences) in non reducing conditions and run under constant voltage (600 V) at 15°C. Molecular weight markers and the Interim Reference Materials ST1 P01/r-hIL-18BP were also loaded onto the gel.

After the electrophoretic run the gel was stained with the Silver Staining Kit-Protein (PlusOne) as described in the instructions contained in the kit leaflet. Briefly, the gel was fixed for 30 minutes in a solution composed of acetic acid and ethanol. After a washing step, the sensitising solution was added and removed after 30 minutes. The gel was washed again and then reacted with the silver solution for 20 minutes. After a washing cycle, the staining was developed in developing solution and subsequently stopped. The gel was then thoroughly washed in water and kept in preserving solution before final storage in Cellophane Sheets.

Gels were scanned and data elaborated using the Phoretix 1 D full software.

### SDS-PAGE AND WESTERN BLOTTING

Two hundred nanograms of r-hlL-18BP were loaded onto the precast gel ExcelGel^{®} SDS Homogeneous 12.5% (by Amersham Biosciences) in non reducing conditions and run under constant voltage (600 V) at 15°C. Molecular weight markers and the Interim Reference Materials ST1 P01/r-hIL-18BP were also loaded onto the gel.

After the electrophoretic run, proteins were transferred from the gel onto a nitrocellulose membrane by passive contact for 60 minutes at room temperature and probed with 0.1 µg/mL of the monoclonal antibody to r-hlL-18BP clone 582.10 (IPL). The reaction was revealed by a chemiluminescent substrate (ECL kit from Amersham Biosciences) after reaction with 1:2000 diluted goat anti-mouse IgG HRP conjugate. The light emission was detected by 10 seconds or 1 minute of exposure to a sensitive autoradiography film.

Coomassie blue or silver staining methods were adopted to detect the MW markers.

After the immunodetection, the film was scanned and the molecular weight (MW) values of the bands were automatically derived from the MW calibration curve using the Phoretix 1-D Full software.

### KG-1 CELLS IN VITRO BIOASSAY

The biological activity of samples was quantified by using an in vitro bioassay. This bioassay was based on the ability of the human acute myelogenous leukemia cell line KG-1 to produce IFN-γ in response to human IL-18 plus human TNF-α in a dose-dependent manner. The r-hlL-18BP specifically binds r-hIL-18 neutralizing its biological activity thereby suppressing the production of IFN-γ.

Briefly, KG-1 cells at 1×10⁵ cells/well were added to a 96 well plate already containing different concentrations of r-hlL-18BP in the presence of a. fixed concentration of r-hIL18 (40 ng/ml in the well) plus a fixed concentration of r-hTNF-α (10 ng/ml in the well). The concentration of each of these two substances combined together was able to give the sub-maximal induction of production of IFN-γ on KG-1 cells. After 24 hr at 37°C, 5% CO₂, the plate was put at -20°C in order to submit the treated cells to a freeze/thaw cycle before performing the immunoassay to determine the quantity of IFN-γ present in the cell supernatant. The cell supernatants was collected and human IFN-γ measured by means of a specific immunoassay (ELISA h-IFN-γ, Duo Set R&D Systems kit). The amount of IFN-γ produced by the treated cells (either with standard curve or IL-18BP sample) was calculated by interpolating the y values (O.D.) on the IFN-γ Standard curve, provided with the kit, fitted by a Sigmoidal dose-response (4PL) Log/Log transformed, thus obtaining the x values (IFN-γ concentrations) (GraphPad Prism).

The biological activity of IL-18BP sample was determined vs the reference preparation by testing the sample at two concentrations falling in the linear part of the reference dose-response curve. At least two independent experiments were carried out. In each independent assay, each concentration was tested in dependent duplicates in a plate.

The titer of IL-18BP sample for each concentration tested, was calculated by interpolating the averaged (two replicates) y values (O.D.) of the amount of IFN-γ produced on the linear part of the reference dose-response curve (Log/Log transformed) thus obtaining the x values (IL-18BP activity).

The value obtained from each concentration was averaged and the final activity of IL-18BP drug substance sample was given by the arithmetic mean of the potencies obtained from each of the independent assay performed.

The titer of the different IL-18BP drug substances was calculated versus the interim Reference Material ST1 P01/r-hIL-18BP.

Two independent experiments were carried out.

### RESULTS

### Background

The primary structure of full length r-hlL-18BP is shown in Fig. 1. The protein has a C-terminal heterogeneity, with molecules ending at residue 164 (full length) and residue 163 (C-1aa), the latter being the main form. Mass spectrometric analysis of tryptic peptides has further shown that the molecule is highly glycosylated, carrying both N- and O-linked oligosaccharides.

The molecule contains four potential N-glycosylation sites, at Asn 49, Asn 64, Asn 73 and Asn 117. Only three of the four sites have been found glycosylated, i.e. Asn 49, Asn 73 and Asn 117, whereas Asn 64 has been found glycosylated only in trace amounts.

The average molecular weight of the whole molecule as determined by SDS-PAGE and SE-HPLC is approximately 50kDa.

The amino acid composition may be taken from table 4.

**TABLE 4 Amino acid composition**

| | Three | letter | | |
|---|---|---|---|---|
| Amino acid | code | Single letter code | No | % |
| Alanine | Ala | A | 13 | 7.9% |
| Arginine | Arg | R | 7 | 4.3% |
| Asparagine | Asn | N | 4 | 2.4% |
| Aspartic acid | Asp | D | 2 | 1.2% |
| Cysteine | Cys | C | 6 | 3.7% |
| Glutamine | Gln | Q | 12 | 7.3% |
| Glutamic acid | Glu | E | 9 | 5.5% |
| Glycine | Gly | G | 9 | 5.5% |
| Histidine | His | H | 4 | 2.4% |
| Isoleucine | Ile | I | 2 | 1.2% |
| Leucine | Leu | L | 19 | 11.6% |
| Lysine | Lys | K | 3 | 1.8% |
| Methionine | Met | M | 0 | 0.0% |
| Phenylalanine | Phe | F | 5 | 3.0% |
| Proline | Pro | P | 17 | 10.4% |
| Serine | Ser | S | 18 | 11.0% |
| Threonine | Thr | T | 15 | 9.1% |
| Tryptophan | Trp | W | 4 | 2.4% |
| Tyrosine | Tyr | Y | 1 | 0.6% |
| Valine | Val | V | 14 | 8.5% |

The routine QC tests of batches from serum-free production revealed that:
- There was a non-conform peptide mapping profile (one major additional peak already during purification of reduced and alkylated protein);
- An abnormal SE-HPLC profile was obtained;
- A double band was detected in SDS-PAGE
- A similar profile was obtained in RP-HPLC
- The specific activity versus homogeneous IL-18BP produced in serum-containing medium (the "reference standard") was comparable.

### PEPTIDE MAPPING PROCEDURE

Since r-hlL-18BP is a highly glycosylated molecule, presenting a high heterogeneity in terms of glycosylation, the protein was submitted to Neuraminidase treatment in order to reduce oligosaccharide heterogeneity due to sialic acid. The protein was then submitted to reduction, carboxymethylation and purification in order to render the trypsin cleavage sites well accessible to the enzyme.

The peptide procedure was carried out with the following steps:

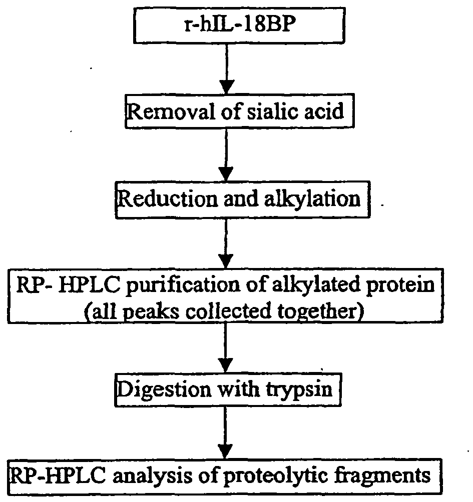

A chromatographic profile different to the one of r-hIL-18BP produced in serum-containing medium, was already detected during the purification of the reduced and alkylated r-hIL-18BP batches from serum-free production (not shown).

Furthermore, the peptide mapping profile of a truncated form of r-hlL-18BP, compared to the current reference standard r-hlL-18BP, showed both an extra peak and a different relative intensity of glycosylated peptides (not shown).

### N-TERMINAL ANALYSIS

The sequence analysis of the intact molecule showed different fragments corresponding to molecule starting from residues 1, 16, 31, and in lower amounts from residues 69,70,107 and 125. The N-terminal analysis is depicted in Fig. 1.

### MALDI-TOF

The spectra obtained by MALDI-TOF showed an additional peak at lower molecular weight (not shown).

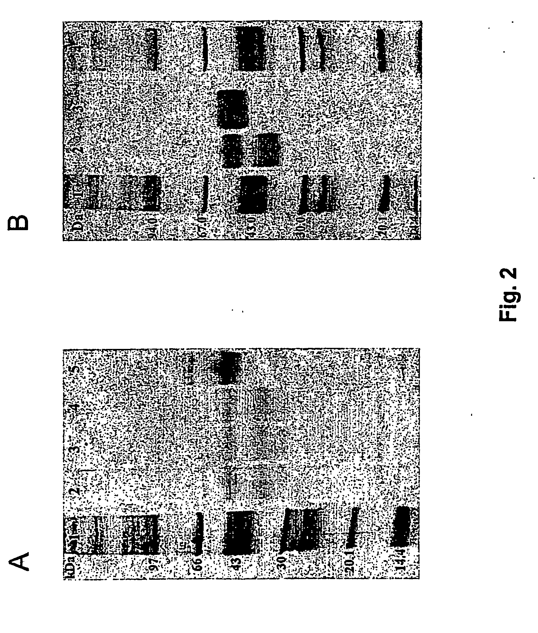

### SDS-PAGE analysis (Fig. 2)

The r-hIL-18BP has a relative molecular weight of about 50 kDa as assigned by 12.5% SDS-PAGE. Serum-free produced r-hlL-18BP showed an additional band of about 40 kDa detected by silver staining. Both bands reacted with an IL-18BP-specific antibody (clone 582.10) in Western Blotting analysis. The silver stained SDS-PAGE gel is depicted in Fig. 2A, the Western Blot in Fig. 2B.

The lanes were occupied as follows:

| | |
|---|---|
| **6.** MW marker | **5.** MW marker |
| **7.** r-hlL-18BP CT20 (2 µg) | **6.** r-hlL-18BP CT20 (200 ng) |
| **8.** r-hlL-18BP CT20 (2 µg) | **7.** ST1P01/r-hlL-18BP (200 ng) |
| **9.** r-hlL-18BP CT20 (2 µg) | |
| **10.** ST1 P01/r-hIL-18BP | **8.** MW marker |

CT20 is a batch of truncated IL-18BP, while ST1 P01 is the standard full-length IL-18BP without truncated forms.

### BIOASSAY

It was assessed whether The results of specific activity of the different IL-18 BP drug substance batches are reported in table 5 where the untruncated (ILNCT16-18 and ST1PO1)and truncated form (highlighted, ILNCT 19-22) are shown.

**TABLE 5**

| **IL-18BP bulks** | **Biological activity U/mL** | **Protein content by O.D. mg/mL** | **Specific activity U/mg** |
|---|---|---|---|
| ILNCT16 | 1,005,906 | 60.3 | 16,682 |
| ILNCT17 | 1,167,546 | 56.8 | 20,555 |
| ILNCT18 | 1,150,841 | 55.3 | 20,811 |
| **ILNCT19** | **949,440** | **61.6** | **15,413** |
| **ILNCT20** | **1,225,693** | **57.2** | **21,428** |
| **ILNCT21** | **1,278,583** | **62.8** | **20,360** |
| **ILNCT22** | **1,347,902** | **59.8** | **22,540** |
| ILNCT23 | 1,200,463 | 60.7 | 19,777 |
| ILNCT24 | 1,013,834 | 56.65 | 17,896 |
| ST1PO1 | 895,69 | 46.39 (AAA) | 19,312 |

This experiment shows that truncated IL-18BP has a biological activity comparable to untruncated IL-18BP-.

### TRUNCATED R-HIL-18BP

In order to characterize the extra peak detected by different techniques, the SE-HPLC analysis was employed to separate the peaks of interest so as to submit them to further characterization steps. For the intended purpose the two peaks were collected separately.

In order to unequivocally identify the collected peaks, peak 1 and peak 2 were re-injected onto the HPLC column.

The two peaks were submitted to peptide mapping according to the protocol described above.

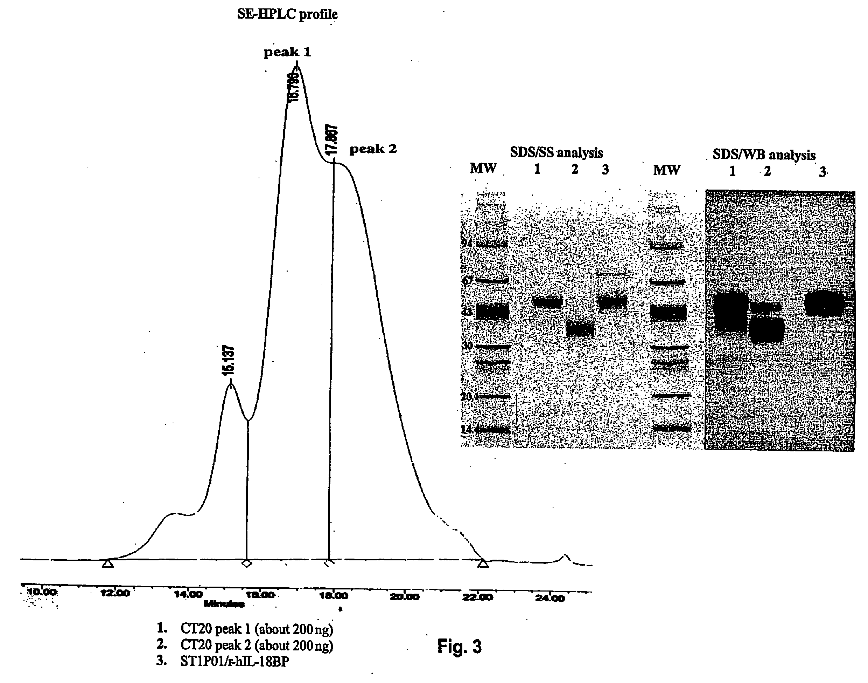

The chromatographic profiles of reduced and alkylated samples are reported in Fig. 3.

Only the main peaks of each fraction were submitted to peptide mapping and analysed by LC-ES/MS:

The extra peak (peptide 31-61) appearing in the peptide mapping profiles is due to internal cleavages of the molecule, as confirmed by the sequence analysis of the peaks and of the intact molecule.

Moreover the different intensities of glycosylated peptides (Pep. 1-15, Pep. 1-32 and Pep. 16-32) show a different glycosylation pattern.

The N-terminal analysis carried out onto peak 1 and peak 2 collected directly from

SE-HPLC analysis, gave the following results:
**N-terminal analysis of Peak 1 isolated by SE-HPLC**

| | | | | | | | | |
|---|---|---|---|---|---|---|---|---|
| N-term | T | P | V | S | Q | X | X | roughly 54% |
| From 31 | A | K | Q | X | P | A | L | roughly 46% |
| From 16 | S | T | K | D | P | C | P | trace |

**N-terminal analysis of Peak 2 isolated by SE-HPLC**

| | | | | | | | | |
|---|---|---|---|---|---|---|---|---|
| **From 16** | S | T | K | D | P | C | P | roughly 63% |
| **From 3J** | A | K | Q | X | P | A | L | roughly 37% |
| **N-term** | T | P | V | S | Q | X | X | trace |

The apparent molecular weight assigned by SDS-PAGE (Fig. 3) was confirmed by MALDI-TOF spectra (not shown).

### CONCLUSIONS

The results obtained employing different analytical tools showed that the following major cleavage sites can be identified:
- Protein truncated at residue 15, i.e. the sequence starting from residue 16 and ending at residue 163/164;
- Protein cleaved at residue 30, i.e. the full length sequence, from residue 1 to residue 163/164, with an internal clipping between residues 30 and 31, held together by disulfides;
- Protein both truncated at residue 15 and cleaved at residue 30, i.e. the sequence starting from residue 16 and ending at residue 163/164, with an internal clipping between residues 30 and 31, held together by disulfides;

When a truncated form of r-hIL18BP is present, the above results show the following:
- A double band is detected by SDS-PAGE of samples. The two bands are detected both by Silver staining and western blotting.
- The SE-HPLC analysis shows an anomalous profile.
- The RP-HPLC chromatographic profiles of reduced and alkylated samples is different as compared to the one of intact samples.
- The peptide mapping profiles showed an extra peak.
- The N-terminal sequence analysis confirms the presence of truncated forms of the molecule.
- Despite the truncated form is present, the specific activity is comparable to that of the intact r-hIL-18BP.

### REFERENCES

1. Conti, B., J. W. Jahng, C. Tinti, J. H. Son, and T. H. Joh. 1997. Induction of interferon-gamma inducing factor in the adrenal cortex. J. Biol. Chem. 272:2035-2037.
2. DiDonato, J A, Hayakawa, M, Rothwarf, D M, Zandi, E and Karin, M. (1997), Nature 388, 16514-16517.
3. Engelmann, H., D. Novick, and D. Wallach. 1990. Two tumor necrosis factor-binding proteins purified from human urine. Evidence for immunological cross-reactivity with cell surface tumor necrosis factor receptors. J. Biol. Chem. 265:1531-1536.
4. Gracie JA, Robertson SE, McInnes IB J Leukoc Biol 2003 Feb;73(2):213-24
5. Kim SH, Eisenstein M, Reznikov L, Fantuzzi G, Novick D, Rubinstein M, Dinarello CA. Structural requirements of six naturally occurring isoforms of the IL-18 binding protein to inhibit IL-18. Proc Natl Acad Sci U S A 2000;97:1190-1195.
6. Micallef, M. J., T. Ohtsuki, K. Kohno, F. Tanabe, S. Ushio, M. Namba, T. Tanimoto, K. Torigoe, M. Fujii, M. Ikeda, S. Fukuda, and M. Kurimoto. 1996. Interferon-gamma-inducing factor enhances T helper 1 cytokine production by stimulated human T cells: synergism with interleukin-12 for interferon-gamma production. Eur-J-Immunol26:1647-51 issn: 0014-2980.
7. Nakamura K, Okamura H, Wada M, Nagata K, Tamura T. Infect Immun 1989 Feb;57(2):590-5
8. Novick, D, Kim, S-H, Fantuzzi, G, Reznikov, L, Dinarello, C, and Rubinstein, M (1999). Immunity 10,127-136.
9. Okamura H, Nagata K, Komatsu T, Tanimoto T, Nukata Y, Tanabe F, Akita K, Torigoe K, Okura T, Fukuda S, et al. Infect Immun 1995 Oct;63(10):3966-72
10. Rothe H, Jenkins NA, Copeland NG, Kolb H. J Clin Invest 1997 Feb 1;99(3):469-74
11. Yoshimoto T, Takeda, K, Tanaka, T, Ohkusu, K, Kashiwamura, S, Okamura, H, Akira, S and Nakanishi, K (1998), J. Immunol. 161, 3400-3407.
12. Xiang and Moss, J. Biol. Chem. 2001 276: 17380-6
13. Xiang and Moss, J. Virol. 2001 75 (20), 9947-54

## Claims

1. An IL-18BP comprising a first polypeptide consisting of amino acids 1 to 30 or of amino acids 15 to 30 of SEQ ID NO: 1 and a second polypeptide consisting of amino acids 31 to 164 or of amino acids 31 to 163 of SEQ ID NO: 1, wherein the first and second polypeptide are linked by a disulfide bond; or a functional derivative, fusion protein or salt thereof, wherein the functional derivative comprises at least one moiety attached to one or more functional groups, which occur as one or more side chains on the amino acid residues.

2. The IL-18BP according to claim 1, wherein the fused protein comprises an immunoglobulin fusion.

3. The IL-18BP according to claim 1, wherein the moiety is a polyethylene glycol (PEG) moiety.

4. Process for production of an IL-18BP according to claim 1 or 2 comprising the step of culturing a host cell comprising a nucleic acid coding for an IL-18BP according to claims 1 or 2 under conditions suitable for expression of said IL-18BP.

5. Process for production of an IL-18BP according to claim 1 or 2 comprising the step of isolating the IL-18BP from the cell culture supernatant of a host cell comprising a nucleic acid coding for an IL-18BP according to claims 1 or 2.

6. Composition comprising an IL-18BP according to any of claims 1 to 3.

7. Use according of IL-18 BP according to any one of claims 1 to 3, for the preparation of a medicament for treatment and/or prevention of a disease selected from: tumor metastasis, psoriasis, arthritis, in particular rheumatoid arthritis, inflammatory bowel disease, Crohn's disease, liver injury, atherosclerosis, sepsis, myocardial infarction, traumatic brain injury, allergy, peripheral vascular disease, multiple sclerosis.

8. The use according to claim 7, wherein the medicament further comprises an interferon, for simultaneous, sequential or separate use.

9. The use according to claim 8, wherein the interferon is interferon-β.

10. The use according to claim 7, wherein the medicament further comprises an inhibitor of Tumor Necrosis Factor (TNF) for simultaneous, sequential or separate use.

11. The use according to claim 10, wherein the inhibitor of TNF is a soluble TNF receptor.

12. The use according to claims 7 to 11, wherein IL-18BP is used in an amount of about 0.001 to 1000 mg/kg of body weight, or about 0.01 to 100 mg/kg of body weight or about 0.1 to 10 mg/kg of body weight or about 5 mg/kg of body weight.

13. The use according to any of claims 7 to 12, wherein the medicament is for subcutaneous administration.

14. The use according to any of claims 7 to 12, wherein medicament is for intramuscular administration.

15. Use of an expression vector comprising a nucleic acid coding for an IL-18BP according to claim 1 or 2 for the manufacture of a medicament for
the treatment and/or prevention of a disease selected from: tumor metastasis, psoriasis, arthritis, in particular rheumatoid arthritis, inflammatory bowel disease. Crohn's disease, liver injury, atherosclerosis, sepsis, myocardial infarction, traumatic brain injury, allergy, peripheral vascular disease, multiple sclerosis.

16. Use of a cell that has been genetically modified to produce an IL-18BP according to claim 1 or 2 for the manufacture of a medicament for the treatment and/or prevention of a disease selected from: tumor metastasis, psoriasis, arthritis, rheumatoid arthritis, inflammatory bowel disease, Crohn's disease, liver injury, atherosclerosis, sepsis, myocardial infarction, traumatic brain injury, allergy, peripheral vascular disease, multiple sclerosis.

## Patentansprüche

1. IL-18BP umfassend ein erstes Polypeptid, das aus den Aminosäuren 1 bis 30 oder aus den Aminosäuren 15 bis 30 von SEQ ID NO:1 besteht, und ein zweites Polypeptid, das aus den Aminosäuren 31 bis 164 oder aus den Aminosäuren 31 bis 163 von SEQ ID NO:1 besteht, wobei das erste und zweite Polypeptid über eine Disulfidbindung verknüpft sind; oder ein funktionelles Derivat, Fusionsprotein oder Salz davon, wobei das funktionelle Derivat mindestens einen Rest umfasst, der an eine oder mehrere funktionelle Gruppen angehängt ist, die als eine oder mehrere Seitenketten auf den Aminosäureresten auftreten.

2. IL-18BP nach Anspruch 1, wobei das fusionierte Protein eine Immunglobulin-Fusion umfasst.

3. IL-18BP nach Anspruch 1, wobei der Rest ein Polyethylenglykol(PEG)-Rest ist.

4. Verfahren zur Herstellung eines IL-18BP nach Anspruch 1 oder 2 umfassend den Schritt Kultivieren einer Wirtszelle, die eine Nukleinsäure umfasst, die für ein IL-18BP nach Ansprüchen 1 oder 2 kodiert, unter Bedingungen, die für die Expression des IL-18BP geeignet sind.

5. Verfahren zur Herstellung eines IL-18BP nach Anspruch 1 oder 2 umfassend den Schritt Isolieren des IL-18BP aus dem Zellkulturüberstand einer Wirtszelle, die eine Nukleinsäure umfasst, die für ein IL-18BP nach Ansprüchen 1 oder 2 kodiert.

6. Zusammensetzung umfassend ein IL-18BP nach einem der Ansprüche 1 bis 3.

7. Verwendung von IL-18BP nach einem der Ansprüche 1 bis 3 zur Herstellung eines Arzneimittels zur Behandlung und/oder Prävention eine Erkrankung, die ausgewählt ist aus: Tumormetastase, Psoriasis, Arthritis, insbesondere rheumatoider Arthritis, entzündlicher Darmerkrankung, Morbus Crohn, Leberverletzung, Atherosklerose, Sepsis, Myokardinfarkt, Schädel-Him-Trauma, Allergie, peripherer Gefäßerkrankung, multipler Sklerose.

8. Verwendung nach Anspruch 7, wobei das Arzneimittel weiter ein Interferon zur gleichzeitigen, sequenziellen oder separaten Verwendung umfasst.

9. Verwendung nach Anspruch 8, wobei das Interferon Interferon-β ist.

10. Verwendung nach Anspruch 7, wobei das Arzneimittel weiter einen Inhibitor von Tumomekrosefaktor (TNF) zur gleichzeitigen, sequenziellen oder separaten Verwendung umfasst.

11. Verwendung nach Anspruch 10, wobei der TNF-Inhibitor ein löslicher TNF-Rezeptor ist.

12. Verwendung nach einem der Ansprüche 7 bis 11, wobei IL-18BP in einer Menge von etwa 0,001 bis 1000 mg/kg Körpergewicht, oder etwa 0,01 bis 100 mg/kg Körpergewicht, oder etwa 0,1 bis 10 mg/kg Körpergewicht oder etwa 5 mg/kg Körpergewicht verwendet wird.

13. Verwendung nach einem der Ansprüche 7 bis 12, wobei das Arzneimittel für subkutane Verabreichung ist.

14. Verwendung nach einem der Ansprüche 7 bis 12, wobei das Arzneimittel für intramuskuläre Verabreichung ist.

15. Verwendung eines Expressionsvektors umfassend eine Nukleinsäure, die für ein IL-18BP nach Anspruch 1 oder 2 kodiert, zur Herstellung eines Medikaments zur Behandlung und/oder Prävention einer Erkrankung, die ausgewählt ist aus: Tumormetastase, Psoriasis, Arthritis, insbesondere rheumatoider Arthritis, entzündlicher Darmerkrankung, Morbus Crohn, Leberverletzung, Atherosklerose, Sepsis, Myokardinfarkt, Schädel-Him-Trauma, Allergie, peripherer Gefäßerkrankung, multipler Sklerose.

16. Verwendung einer Zelle, die genetisch modifiziert worden ist, um ein IL-18BP nach Anspruch 1 oder 2 zu produzieren, zur Herstellung eines Arzneimittels zur Behandlung und/oder Prävention einer Erkrankung, die ausgewählt ist aus: Tumormetastase, Psoriasis, Arthritis, insbesondere rheumatoider Arthritis, entzündlicher Darmerkrankung, Morbus Crohn, Leberverletzung, Atherosklerose, Sepsis, Myokardinfarkt, Schädel-Him-Trauma; Allergie, peripherer Gefäßerkrankung, multipler Sklerose.

## Revendications

1. Protéine IL-18BP comprenant un premier polypeptide consistant en les acides aminés 1 à 30 ou en les acides aminés 15 à 30 de la Séquence n° 1 et un second polypeptide consistant en les acides aminés 31 à 164 ou en les acides aminés 31 à 163 de la Séquence n° 1, les premier et second polypeptides étant reliés par un pont disulfure ; ou dérivé fonctionnel, protéine de fusion d'une telle protéine, le dérivé fonctionnel comprenant au moins un fragment attaché à un ou plusieurs groupes fonctionnels qui se présentent sous forme d'une ou plusieurs chaînes latérales sur les résidus acide aminé.

2. Protéine IL-18BP selon la revendication 1, dans laquelle la protéine fusionnée comprend une fusion d'immunoglobuline.

3. Protéine IL-18BP selon la revendication 1, dans laquelle le fragment est un fragment polyéthylèneglycol (PEG).

4. Procédé pour la production d'une protéine IL-18BP selon la revendication 1 ou 2, comprenant l'étape de culture d'une cellule hôte comprenant un acide nucléique codant une protéine IL-18BP selon la revendication 1 ou 2, dans des conditions appropriées à l'expression de ladite protéine IL-18BP.

5. Procédé pour la production d'une protéine IL-18BP selon la revendication 1 ou 2, comprenant l'étape d'isolement de la protéine IL-18BP à partir du surnageant de culture cellulaire d'une cellule hôte comprenant un acide nucléique codant une protéine IL-18BP selon la revendication 1 ou 2.

6. Composition comprenant une protéine IL-18BP selon l'une quelconque des revendications 1 à 3.

7. Utilisation de la protéine IL-18BP selon l'une quelconque des revendications 1 à 3, pour la fabrication d'un médicament destiné au traitement et/ou à la prévention d'une maladie choisie parmi : la dissémination métastatique de tumeurs, le psoriasis, l'arthrite, en particulier la polyarthrite rhumatoïde, la maladie intestinale inflammatoire, la maladie de Crohn, une atteinte hépatique, l'athérosclérose, le sepsis, l'infarctus du myocarde, une atteinte cérébrale traumatique, l'allergie, les maladies vasculaires périphériques, la sclérose en plaques.

8. Utilisation selon la revendication 7, dans laquelle le médicament comprend en outre un interféron, pour emploi simultané, séquentiel ou séparé.

9. Utilisation selon la revendication 8, dans laquelle l'interféron est l'interféron β.

10. Utilisation selon la revendication 7, dans laquelle le médicament comprend en outre un inhibiteur du facteur de nécrose tumorale (TNF), pour emploi simultané, séquentiel ou séparé.

11. Utilisation selon la revendication 10, dans laquelle l'inhibiteur de TNF est un récepteur de TNF soluble.

12. Utilisation selon les revendications 7 à 11, dans laquelle la protéine IL-18BP est utilisée en une quantité d'environ 0,001 à 1 000 mg/kg de poids corporel d'environ 0,01 à 100 mg/kg de poids corporel ou d'environ 0,1 à 10 mg/kg de poids corporel ou d'environ 5 mg/kg de poids corporel.

13. Utilisation selon l'une quelconque des revendications 7 à 12, dans laquelle le médicament est destiné à l'administration par voie sous-cutanée.

14. Utilisation selon l'une quelconque des revendications 7 à 12, dans laquelle le médicament est destiné à l'administration par voie intramusculaire.

15. Utilisation d'un vecteur d'expression comprenant un acide nucléique codant une protéine IL-18BP selon la revendication 1 ou 2, pour la fabrication d'un médicament destiné au traitement et/ou à la prévention d'une maladie choisie parmi : la dissémination métastatique de tumeurs, le psoriasis, l'arthrite, en particulier la polyarthrite rhumatoïde, la maladie intestinale inflammatoire, la maladie de Crohn, une atteinte hépatique, l'athérosclérose, le sepsis, l'infarctus du myocarde, une atteinte cérébrale traumatique, l'allergie, les maladies vasculaires périphériques, la sclérose en plaques.

16. Utilisation d'une cellule qui a été génétiquement modifiée pour produire une protéine IL-18BP selon la revendication 1 ou 2, pour la fabrication d'un médicament destiné au traitement et/ou à la prévention d'une maladie choisie parmi : la dissémination métastatique de tumeurs, le psoriasis, l'arthrite, la polyarthrite rhumatoïde, la maladie intestinale inflammatoire, la maladie de Crohn, une atteinte hépatique, l'athérosclérose, le sepsis, l'infarctus du myocarde, une atteinte cérébrale traumatique, l'allergie, les maladies vasculaires périphériques, la sclérose en plaques.


### SEQUENCE LISTING

<110> Applied Research Systems ARS Holding N.V:
<120> Active fragments of the IL-18 binding protein
<130> 818 WO
<160> 7
<170> PatentIn version 3.1
<210> 1
   <211> 164
   <212> PRT
   <213> homo sapiens
<400> 1
   Thr Pro Val Ser Gln Thr Thr Thr Ala Ala Thr Ala Ser Val Arg Ser
   1               5                   10                  15
   Thr Lys Asp Pro Cys Pro Ser Gln Pro Pro Val Phe Pro Ala Ala Lys
               20                  25                  30
   Gln Cys Pro Ala Leu Glu Val Thr Trp Pro Glu Val Glu Val Pro Leu
           35                  40                  45
   Asn Gly Thr Leu Ser Leu Ser Cys Val Ala Cys Ser Arg Phe Pro Asn
       50                  55                  60
   Phe Ser Ile Leu Tyr Trp Leu Gly Asn Gly Ser Phe Ile Glu His Leu
   65 70 75 80
   Pro Gly Arg Leu Trp Glu Gly Ser Thr Ser Arg Glu Arg Gly Ser Thr
   85 90 95
   Gly Thr Gln Leu Cys Lys Ala Leu Val Leu Glu Gln Leu Thr Pro Ala
   100 105 110
   Leu His Ser Thr Asn Phe Ser Cys Val Leu Val Asp Pro Glu Gln Val
   115 120 125
   Val Gln Arg His Val Val Leu Ala Gln Leu Trp Ala Gly Leu Arg Ala
   130 135 140
   Thr Leu Pro Pro Thr Gln Glu Ala Leu Pro Ser Ser His Ser Ser Pro
   145 150 155 160
   Gln Gln Gln Gly
<210> 2
   <211> 149
   <212> PRT
   <213> homo sapiens
<400> 2
   Ser Thr Lys Asp Pro Cys Pro Ser Gln Pro Pro Val Phe Pro Ala Ala
   1 5 10 15
   Lys Gln Cys Pro Ala Leu Glu Val Thr Trp Pro Glu Val Glu Val Pro
   20 25 30
   Leu Asn Gly Thr Leu Ser Leu Ser Cys Val Ala Cys Ser Arg Phe Pro
   35 40 45
   Asn Phe Ser Ile Leu Tyr Trp Leu Gly Asn Gly Ser Phe Ile Glu His
   50 55 60
   Leu Pro Gly Arg Leu Trp Glu Gly Ser Thr Ser Arg Glu Arg Gly Ser
   65 70 75 80
   Thr Gly Thr Gln Leu Cys Lys Ala Leu Val Leu Glu Gln Leu Thr Pro
   85 90 95
   Ala Leu His Ser Thr Asn Phe Ser Cys Val Leu Val Asp Pro Glu Gln
   100 105 110
   Val Val Gln Arg His Val Val Leu Ala Gln Leu Trp Ala Gly Leu Arg
   115 120 125
   Ala Thr Leu Pro Pro Thr Gln Glu Ala Leu Pro Ser Ser His Ser Ser
   130 135 140
   Pro Gln Gln Gln Gly
   145
<210> 3
   <211> 134
   <212> PRT
   <213> homo sapiens
<400> 3
   Ala Lys Gln Cys Pro Ala Leu Glu Val Thr Trp Pro Glu Val Glu Val
   1 5 10 15
   Pro Leu Asn Gly Thr Leu Ser Leu Ser Cys Val Ala Cys Ser Arg Phe
   20 25 30
   Pro Asn Phe Ser Ile Leu Tyr Trp Leu Gly Asn Gly Ser Phe Ile Glu
   35 40 45
   His Leu Pro Gly Arg Leu Trp Glu Gly Ser Thr Ser Arg Glu Arg Gly
   50 55 60
   Ser Thr Gly Thr Gln Leu Cys Lys Ala Leu Val Leu Glu Gln Leu Thr
   65 70 75 80
   Pro Ala Leu His Ser Thr Asn Phe Ser Cys Val Leu Val Asp Pro Glu
   85 90 95
   Gln Val Val Gln Arg His Val Val Leu Ala Gln Leu Trp Ala Gly Leu
   100 105 110
   Arg Ala Thr Leu Pro Pro Thr Gln Glu Ala Leu Pro Ser Ser His Ser
   115 120 125
   Ser Pro Gln Gln Gln Gly
   130
<210> 4
   <211> 96
   <212> PRT
   <213> homo sapiens
<400> 4
   Tyr Trp Leu Gly Asn Gly Ser Phe Ile Glu His Leu Pro Gly Arg Leu
   1 5 10 15
   Trp Glu Gly Ser Thr Ser Arg Glu Arg Gly Ser Thr Gly Thr Gln Leu
   20 25 30
   Cys Lys Ala Leu Val Leu Glu Gln Leu Thr Pro Ala Leu His Ser Thr
   35 40 45
   Asn Phe Ser Cys Val Leu Val Asp Pro Glu Gln Val Val Gln Arg His
   50 55 60
   Val Val Leu Ala Gln Leu Trp Ala Gly Leu Arg Ala Thr Leu Pro Pro
   65 70 75 80
   Thr Gln Glu Ala Leu Pro Ser Ser His Ser Ser Pro Gln Gln Gln Gly
   85 90 95
<210> 5
   <211> 95
   <212> PRT
   <213> homo sapiens
<400> 5
   Trp Leu Gly Asn Gly Ser Phe Ile Glu His Leu Pro Gly Arg Leu Trp
   1 5 10 15
   Glu Gly Ser Thr Ser Arg Glu Arg Gly Ser Thr Gly Thr Gln Leu Cys
   20 25 30
   Lys Ala Leu Val Leu Glu Gln Leu Thr Pro Ala Leu His Ser Thr Asn
   35 40 45
   Phe Ser Cys Val Leu Val Asp Pro Glu Gln Val Val Gln Arg His Val
   50 55 60
   Val Leu Ala Gln Leu Trp Ala Gly Leu Arg Ala Thr Leu Pro Pro Thr
   65 70 75 80
   Gln Glu Ala Leu Pro Ser Ser His Ser Ser Pro Gln Gln Gln Gly
   85 90 95
<210> 6
   <211> 58
   <212> PRT
   <213> homo sapiens
<400> 6
   Glu Gln Leu Thr Pro Ala Leu His Ser Thr Asn Phe Ser Cys Val Leu
   1 5 10 15
   Val Asp Pro Glu Gln Val Val Gln Arg His Val Val Leu Ala Gln Leu
   20 25 30
   Trp Ala Gly Leu Arg Ala Thr Leu Pro Pro Thr Gln Glu Ala Leu Pro
   35 40 45
   Ser Ser His Ser Ser Pro Gln Gln Gln Gly
   50 55
<210> 7
   <211> 40
   <212> PRT
   <213> homo sapiens
<400> 7
   Pro Glu Gln Val Val Gln Arg His Val Val Leu Ala Gln Leu Trp Ala
   1 5 10 15
   Gly Leu Arg Ala Thr Leu Pro Pro Thr Gln Glu Ala Leu Pro Ser Ser
   20 25 30
   His Ser Ser Pro Gln Gln Gln Gly
   35 40